# FASCICULE DE BREVET EUROPEEN

(11) **EP 1 611 246 B1**
(45) Date de publication et mention de la délivrance du brevet: **02.06.2010**
(21) Numéro de dépôt: 04742827.1
(22) Date de dépôt: 02.04.2004
(51) Int. Cl.: C12Q 1/04, C12Q 1/10

(54) **MILIEU DE DETECTION ET/OU D' IDENTIFICATION DE MICRO-ORGANISMES**
IDENTIFIZIERUNG- UND NACHWEISMITTEL VON MIKROORGANISMEN
MEDIUM FOR THE DETECTION AND IDENTIFICATION OF MICRO-ORGANISMS

(30) Priorité: 07.04.2003 FR 0304263
(43) Date de publication de la demande: 04.01.2006
(73) Titulaire: BIOMERIEUX, 69280 Marcy-L'Etoile (FR)
(72) Inventeur: ORENGA, Sylvain, F-01600 Neuville sur Ain (FR); ROGER-DALBERT, Céline, F-01150 Vaux en Bugey (FR); PERRY, John, Newcastle-Upon-Tyne NE2HR (GB); JAMES, Arthur, Cockermouth Cumbria CA13 9UX (GB)
(86) Numéro de dépôt international: PCT/FR2004/050139
(87) Numéro de publication internationale: WO 2004/092400

(56) Documents cités:
- EP-A- 0 326 635
- EP-A- 0 881 284
- WO-A-99/09207
- FR-A- 2 697 028
- SONG XIAOPING ET AL: "Synthesis and stability study of a modified phenylpropionic acid linker-based esterase-sensitive prodrug." BIOORGANIC AND MEDICINAL CHEMISTRY LETTERS, vol. 12, no. 23, 2 décembre 2002 (2002-12-02), pages 3439-3442, XP001204115 ISSN: 0960-894X
- GRAVIER-PELLETIER C ET AL: "Synthesis and glycosidase inhibitory activity of enantiopure polyhydroxylated octahydroindoles and decahydroquinolines, analogs to castanospermine" TETRAHEDRON, ELSEVIER SCIENCE PUBLISHERS, AMSTERDAM, NL, vol. 59, no. 44, 27 octobre 2003 (2003-10-27), pages 8721-8730, XP004464867 ISSN: 0040-4020

## Description

Le domaine de l'invention est celui de l'analyse microbiologique par voie biochimique, et en particulier de la détection et de l'identification de microorganismes par ensemencement de milieux réactionnels.

Dans le cadre de l'invention, on s'intéresse plus particulièrement à la détection et à l'identification de microorganismes, tels que notamment des bactéries ou des levures, pathogènes ou indicateurs de qualité, que ce soit dans le milieu médical ou le milieu industriel.

Il existe actuellement de très nombreux milieux permettant la détection de ces microorganismes. Cette détection peut être basée notamment sur l'utilisation de substrats particuliers, spécifiques d'une enzyme du microorganisme que l'on souhaite détecter, comme le décrit par exemple le brevet EP 0 326 635. D'une manière générale, les substrats synthétiques d'enzymes sont constitués d'une première partie spécifique de l'activité enzymatique à révéler, et d'une seconde partie faisant office de marqueur, généralement chromogène ou fluorescent. Par le choix de ces substrats, selon qu'il y a réaction ou non, il est possible de caractériser la nature d'un microorganisme. Ainsi, dans le cas de bactéries, les souches d'*Escherichia coli* sont souvent mises en évidence par la révélation d'une activité enzymatique du type osidase telle que l'activité β-glucuronidase ou β-galactosidase. De la même façon, le genre *Listeria* peut être détecté par la mise en évidence d'une activité β-glucosidase.
Une activité aminopeptidase peut également être utilisée pour révéler un groupe, un genre ou une espèce de bactéries. Ainsi, l'activité alanine-aminopeptidase, par exemple, permet de différencier les bactéries à Gram négatif des bactéries à Gram positif.
Enfin, on peut citer également la détection d'une activité estérase pour notamment la mise en évidence du genre *Salmonella.* En effet, le genre *Salmonella* possède des estérases non spécifiques capables d'hydrolyser des substrats synthétiques chromogènes, par exemple indigogéniques. Dans le cas de la détection de salmonelles, et plus généralement dans le cas de bactéries à activité estérase, la détection et/ou l'identification de ces bactéries est classiquement réalisée sur des milieux gélosés ou liquides d'isolement, qui permettent la détection et/ou l'identification des colonies suspectes de bactéries à activité estérase.

Toutefois, on observe lors de certains prélèvements d'échantillons, notamment de selles, la présence d'enzymes, non spécifiques du microorganisme que l'on souhaite détecter, et libres dans l'échantillon (on parle alors d' « enzymes libres »), et qui peuvent ultérieurement réagir avec le substrat chromogène. Ces enzymes « libres » peuvent notamment être présentes dans un prélèvement biologique, tel qu'un échantillon de selles, et provenir de cellules du tractus digestif, du foie, du pancréas et donc se retrouver via ces organes dans le prélèvement biologique. Des enzymes libres peuvent également être présentes dans des échantillons alimentaires que l'on souhaite analyser, tels que par exemples des foies de volaille, dans ce cas les enzymes sont issues des cellules du foie. Cela induit la suspicion de positifs ne pouvant être confirmés : certains prélèvements sont considérés comme contaminés alors qu'ils ne le sont pas, ce qui peut avoir des conséquences dramatiques pour le diagnostic ultérieur. Ainsi, dans le cas de la détection de salmonelles mises en évidence par une activité enzymatique de type estérase, le prélèvement de selles non contaminé par des salmonelles, peut contenir des estérases « libres » qui hydrolysent alors le substrat présent dans le milieu de culture, ce qui provoque la libération d'une coloration magenta, normalement spécifique des salmonelles.
La présente invention se propose donc d'améliorer les milieux permettant la détection de microorganismes actuellement commercialisés en limitant la présence de faux positifs engendrée par la présence d'enzymes libres dans le prélèvement et non spécifique d'un micro-organisme. D'une manière surprenante, les inventeurs ont mis en évidence que l'utilisation de certains composés chimiques inhibaient les enzymes libres de l'échantillon, sans inhiber les enzymes non libres, c'est à dire les enzymes provenant des microorganismes que l'on souhaite détecter.

A cet effet, la présente invention concerne un milieu de détection de microorganismes présents dans un échantillon, tel que notamment un échantillon biologique ou alimentaire comprenant un milieu de culture et au moins un substrat qui peut être hydrolysé en un produit marqué par au moins une première enzyme non libre dans l'échantillon et spécifique desdits microorganismes, **caractérisé en ce qu**'il comprend, en outre au moins un inhibiteur d'au moins une deuxième enzyme, identique à la première mais libre dans ledit échantillon et ne provenant pas d'un microorganisme, ledit inhibiteur étant un composé de formule (I), *infra.*

Au sens de la présente invention, le terme microorganisme recouvre les bactéries, les levures, et plus généralement, les organismes généralement unicellulaire, invisibles à l'oeil nu, qui peuvent être multipliés et manipulés en laboratoire.
Selon un mode préféré de réalisation de l'invention, le microorganisme est une bactérie, gram négative ou positive, ou une levure.
A titre de bactéries Gram négatives, on peut citer les bactéries des genres suivants : *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella*, *Neisseria*, *Burkholderia*, *Citrobacter*, *Hafnia*, *Edwardsiella* et *Legionella.*
A titre de bactéries Gram positives, on peut citer les bactéries des genres suivants : *Enterococcus, Streptococcus, Staphylococcus, Bacillus, Listeria, Clostridium, Mycobacteria* et *Corynebacteria.*
A titre de levures, on peut citer les levures des genres suivants : *Candida, Cryptococcus*, *Saccharomyces* et *Trichosporon.*
Selon un mode préféré de l'invention, le microorganisme est une bactérie, qui appartient préférentiellement au genre *Salmonella,* ou une levure, qui appartient préférentiellement au genre *Candida.*

Au sens de la présente invention, on entend par milieu de culture, un milieu comprenant tous les éléments nécessaires à la survie et/ou à la croissance de microorganismes. En pratique, l'homme du métier choisira le milieu de culture en fonction des microorganismes cibles, selon des critères parfaitement connus et à la portée de cet homme de l'art. Pour les bactéries, on peut citer à titre indicatif, les milieux sélectifs de type : Mac Conkey, Columbia ANC, PALCAM, et les milieux non sélectifs de type Trypcase soja, milieu nutritif. Pour les levures, on peut citer comme milieu de culture Sabouraud Gentamycine-Chloramphénicol, ou Sabouraud.
Le milieu de culture selon l'invention peut contenir d'éventuels autres additifs comme par exemple : des peptones, un ou plusieurs facteurs de croissance, des hydrates de carbone, un ou plusieurs agents sélectifs, des tampons, un ou plusieurs gélifiants... Ce milieu de culture peut se présenter sous forme de liquide de gel prêt à l'emploi, c'est à dire prêt à l'ensemencement en tube, flacon, ou sur boite de Petri.
Au sens de la présente invention, le substrat est choisi parmi tout substrat pouvant être hydrolysé en un produit qui permet la détection, directe ou indirecte d'un microorganisme. Préférentiellement, ce substrat comprend
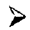 une première partie spécifique de l'activité enzymatique à révéler. Cette première partie est susceptible d'interagir avec ladite première enzyme, qui est spécifique du microorganisme recherché, mais également avec ladite deuxième enzyme,
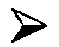 une seconde partie faisant office de marqueur, ci-après appelée partie marqueur, qui peut être fluorescente ou chromogène.
Comme substrat fluorescent, on peut citer notamment les substrats à base d'umbelliférone ou d'aminocoumarine, à base de résorufine ou encore à base de fluorescéïne.
Comme substrat chromogène, mieux adapté aux supports solides (filtre, gélose, gel d'électrophorèse), on peut citer notamment les substrats à base d'indoxyl et ses dérivés, et les substrats à base d'hydroxyquinoline ou d'esculétine et leurs dérivés, qui permettent la détection d'activités osidase et estérase. On peut citer également les substrats à base de nitrophénol et nitroaniline et dérivés, permettant de détecter les activités osidases et estérases dans le cas de substrats à base de nitrophénol, et des activités peptidases dans le cas de substrats à base de la nitroaniline. On peut citer enfin les substrats à base de naphtol et naphtylamine et ses dérivés, qui permettent de détecter les activités osidases et estérases par l'intermédiaire du naphtol, et les activités peptidases par l'intermédiaire de la naphtylamine. Ce substrat peut permettre notamment, mais d'une façon non limitative, la détection d'une activité enzymatique telle que l'activité d'une osidase, peptidase, estérase...

Par échantillon, on entend tout type d'échantillon dans lequel on souhaite détecter la présence de microorganismes. Cet échantillon peut être un échantillon biologique ou alimentaire. Cet échantillon peut provenir notamment, mais de façon non limitative, d'un prélèvement de sang, d'urine, de selles, d'aliments...
Par première enzyme, on entend une estérase non libre dans l'échantillon, c'est à dire provenant du microorganisme que l'on souhaite détecter. Cette enzyme permet l'hydrolyse du substrat en un produit marqué.
Par deuxième enzyme, on entend une enzyme identique à la première mais libre dans l'échantillon, c'est à dire qu'elle ne provient pas du microorganisme recherché. Cette deuxième enzyme est susceptible de réagir avec le substrat, ce qui peut induire la présence de faux positifs.

L'inhibiteur appartient à la famille des organophosphates, et est un composé de formule (I) 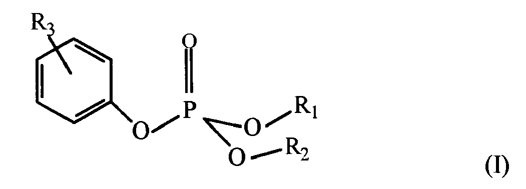 dans laquelle
- R₁ est un atome d'hydrogène, ou un groupement alkyle, aryle, halogène
- R₂ est un atome d'hydrogène ou un groupement alkyle, aryle, halogène
- R₃, est rien ou un groupement alkyle, aryle, NO2
Selon l'invention, on entend notamment par aryle un noyau aromatique en C₆-C₁₀, notamment phényle, benzyle, 1-naphtyle ou 2-naphtyle.
On entend par alkyle un alkyle en C₁-C₆, à savoir un alkyle droit ou ramifié ayant de 1 à 6 atomes de carbone. A titre d'exemple, on peut citer le méthyle, l'éthyle, le propyle, l'iso-propyle, le butyle, le t-butyle, le pentyle, l'iso-pentyle et l'hexyle.

Selon un mode préféré de réalisation de l'invention, l'inhibiteur est un composé de formule (I) dans lequel
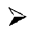 R1 et R2 sont des groupements éthyl
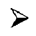 R3 est groupement NO2

Ce composé est le O,O-Diethyl p-nitrophenyl phosphate ayant la formule suivante :

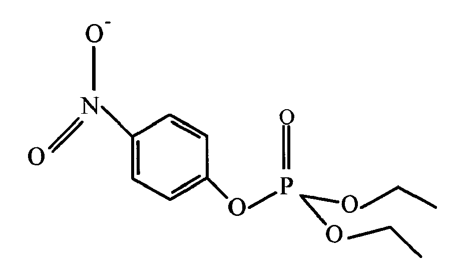

Selon un autre mode préféré de réalisation de l'invention, l'inhibiteur est un composé de formule (I) dans lequel
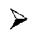 R1 et R2 sont des groupements méthyl
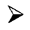 R3 est un groupement NO2

Ce composé est le O,O-Dimethyl p-nitrophenyl phosphate ayant la formule suivante :

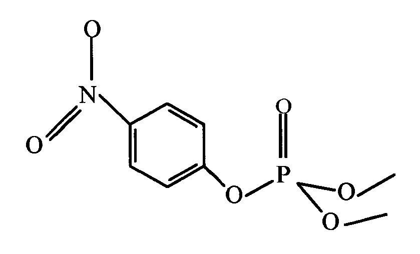

Selon un autre mode préféré de réalisation de l'invention, l'inhibiteur est un composé de formule (I) dans lequel
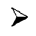 R1 et R2 sont des groupement chloroethyl
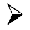 R3 est un cycle de formule

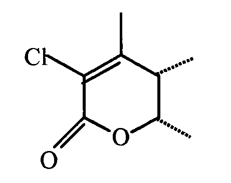

Les lignes pointillées permettre de visualiser la position dudit cycle dans le composé de formule (I) de l'invention.

Ce composé est le le O,O-Di (2-chloroethyl)-O-(3-chloro-4-methylcoumarin-7-YL) phosphate ayant la formule suivante :

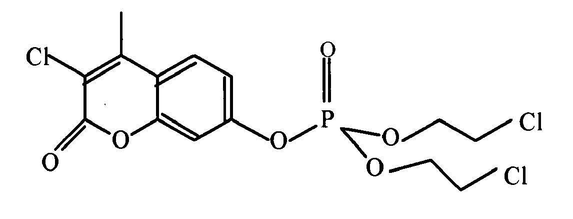

L'inhibiteur peut être également un dérivé des molécules présentées ci dessus.

La concentration en O,O-Diethyl p-nitrophenyl phosphate ou son dérivé dans le milieu de détection est préférentiellement comprise entre 0,1 et 15 mg/l, et encore plus préférentiellement entre 1 et 10 mg/l.
La concentration en O,O-Dimethyl p-nitrophenyl phosphate ou son dérivé dans le milieu de détection est préférentiellement comprise entre 0,1 et 100 mg/l, et encore plus préférentiellement entre 10 et 50 mg/l.
La concentration en O,O-Di(2-chloroethyl)-O-(3-chloro-4-methylcoumarin-7-YL) phosphate ou son dérivé dans le milieu de détection est préférentiellement comprise entre 1 et 1000 mg/l, et encore plus préférentiellement entre 30 et 100 mg/l.

Selon un mode préféré de l'invention, ledit substrat est un substrat chromogène, préférentiellement un ester d'indoxyl ou de ses dérivés, et encore plus préférentiellement un ester d'indoxyl ayant la formule (III) suivante 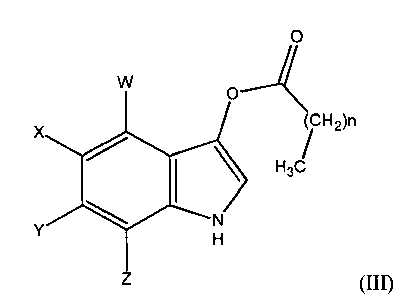 avec n compris entre 1 et 12 et W, X, Y, Z sont choisis parmi H, Br, Cl, F, I.

D'une façon encore plus préférentielle, le substrat est le 5-Bromo-6-chloro-3-indoxyl caprylate (Magenta C8, N°CAS 209347-94-4) ou le 5-Bromo-3-indoxyl nonanoate (Biosynth).

L'invention concerne également un procédé de détection et/ou d'identification de microorganismes comprenant :
- l'ensemencement desdits microorganismes à identifier sur un milieu de détection, tel que définit précédemment,
- l'incubation du milieu de détection ensemencé avec lesdits microorganismes à identifier, et
- la détermination de la présence desdits microorganismes par la détection du ou des substrats hydrolysés en un produit marqué.

L'ensemencement des microorganimes, tels que notamment les bactéries ou levures peut être réalisée par toutes les techniques d'ensemencement bien connues de l'homme du métier. De même, l'incubation est réalisée préférentiellement à une température pour laquelle la croissance des microorganismes et l'activité enzymatique que l'on souhaite détecter sont maximales, que l'homme du métier peut choisir aisément selon l'activité enzymatique à détecter. A titre indicatif, l'incubation est préférentiellement réalisée entre 36 et 38°C.

L'invention concerne l'utilisation du milieu de détection et/ou d'identification tel que défini ci dessus pour identifier des microorganismes.

Les exemples ci dessous sont donnés à titre explicatif et n'ont aucun caractère limitatif. Ils permettront de mieux comprendre l'invention.

### Exemple 1 : Inhibition des estérases libres dans un échantillon, par le O,O-Diethyl p-nitrophenyl phosphate lors de l'identification de bactéries Salmonelles

Le but des expériences présentées dans cet exemple est de démontrer l'effet inhibiteur du O,O-Diethyl p-nitrophenyl phosphate (paraoxon® éthyl ; Riedel-deHaën, St Quentin Fallavier, France) sur des estérases libres (dans un échantillon de selles contaminé par des salmonelles).

Préparation du milieu de détection : un volume de 250 ml de milieu de détection est préparé à partir d'une poudre de milieu ayant la composition suivante :

| | |
|---|---|
| Peptones | 6,25 g/l |
| Tris | 0,16 g/l |
| Lactose | 6 g/l |
| Sels biliaires | 1,5 g/l |
| NaCl | 5 g/l |
| Agar | 14 g/l |

La fonte est réalisée à 100°C, et le milieu est autoclavé 15 minutes à 121°C. On ajoute ensuite les additifs dans le milieu: Magenta C8 (500mg/l; B-7102, BIOSYNTH, Staad, Suisse); X-glucoside (75 mg/l; B-7250, BIOSYNTH, Staad, Suisse); cefsulodine (10 mg/l; C 4786, Sigma, St Quentin Fallavier, France) et différentes concentrations de l'inhibiteur O,O-Diethyl p-nitrophenyl phosphate (Paraoxon® éthyl; CAS n° 311-45-5 ; 36186, Riedel-de Haën, St Quentin Fallavier, France ; 0 ; 1 ; 5 ou 10 mg/l).
Les différents milieux de détection ainsi obtenus sont ensuite coulés en boîte de Petri.

Ensemencement des bactéries *Salmonella* : 10 µl de selles sont déposés sur boîte de Petri, cette suspension de selle est mélangée ou non à 10µl d'une suspension de *Salmonella* (0,5 McF) et isolée en 3 cadrans sur une boîte de Petri. Différentes selles et différentes souches de *Salmonella* provenant de la collection de la demanderesse sont utilisées selon ce protocole. Les boîtes de Petri sont incubées 24 heures à 37°C. La lecture de l'apparition d'une coloration au niveau du point de dépôt de la selle (colonne « dépôt ») et au niveau des colonies de *Salmonella* (colonne « colonie ») est effectuée selon une échelle semi-quantitative :
0 = absence de coloration
0,5 = trace de coloration
1 = coloration faible
2 = coloration forte .

Les résultats obtenus sont présentés dans le tableau 1.

**Tableau 1 : Effets de l'inhibiteur O,O-Diethyl p-nitrophenyl phosphate sur l'activité des estérases libres dans un échantillon de selles lors de l'identification de bactéries Salmonella**

| | | [O,O-Diethyl p-nitrophenyl phosphate] en mg/l | | | | | | | |
|---|---|---|---|---|---|---|---|---|---|
| | | 0 | | 1 | | 5 | | 10 | |
| Souche | N° selle | Dépôt | Colonies | Dépôt | Colonies | Dépôt | Colonies | Dépôt | Colonies |
| absence | A | 1 | 0 | 0,5 | 0 | 0 | 0 | 0 | 0 |
| | B | 0 | 0 | 0 | 0 | 0 | 0 | 0 | 0 |
| *Salmonella Enteritidis* | A | 1 | 2 | 0,5 | 2 | 0 | 2 | 0 | 2 |
| | B | 0 | 2 | 0 | 2 | 0 | 2 | 0 | 2 |
| *Salmonella Paratyphi A* | A | 1 | 2 | 0,5 | 1 | 0 | 1 | 0 | 1 |
| | B | 0 | 2 | 0 | 1 | 0 | 1 | 0 | 1 |
| *Salmonella Typhi* | A | 1 | 1 | 0,5 | 1 | 0 | 1 | 0 | 1 |
| | B | 0 | 1 | 0 | 1 | 0 | 1 | 0 | 1 |

Comme le présente le tableau 1, la coloration est diminuée au niveau du point de dépôt de la selle en présence de l'inhibiteur, alors qu'elle est maintenue au niveau des colonies des bactéries que l'inhibiteur soit présent ou absent. Les enzymes libres dans les selles (dénommée dans l'exposé de l'invention « deuxième enzyme libre »), et qui se situent principalement au niveau du point de dépôt de la selle sont donc bien inhibées par l'inhibiteur, alors que les enzymes provenant des bactéries (dénommée dans l'exposé de l'invention « première enzyme non libre »), et qui se situent principalement au niveau des colonies de salmonelles, ne sont pas inhibées. La coloration à l'endroit du dépôt, observé lors du dépôt de selles en l'absence de souche, démontre la présence d'une réaction liée à la présence d'enzymes libres du prélèvement, qui ne proviennent pas de la contamination par des Salmonelles. Ces résultats démontrent que les réactions enzymatiques aspécifiques dues à la présence d' « enzymes libres » sont inhibées fortement en présence de l'inhibiteur O,O-Diethyl p-nitrophenyl phosphate, ce qui limite la détection de faux positifs.

### Exemple 2: Inhibition des estérases libres dans un échantillon, par le O,O-dimethyl p-nitrophenyl phosphate lors de l'identification de bactéries Salmonelles

Le but des expériences présentées dans cet exemple est de démontrer l'effet inhibiteur des estérases libres du O,O-Dimethyl p-nitrophenyl phosphate (paraoxon® méthyl ; Riedel-deHaën, St Quentin Fallavier, France) sur des selles contaminées par des salmonelles.

Préparation du milieu de détection : un volume de 250 ml de milieu de détection est préparé à partir d'une poudre de milieu ayant la composition suivante :

| | |
|---|---|
| Peptones | 6,25 g/l |
| Tris | 0,16 g/l |
| Lactose | 6 g/l |
| Sels biliaires | 1,5 g/l |
| NaCl | 5 g/l |
| Agar | 14 g/l |

La fonte est réalisée à 100°C, et le milieu est autoclavé 15 minutes à 121°C. On ajoute ensuite les additifs dans le milieu : Magenta C8 (500mg/l; B-7102, BIOSYNTH, Staad, Suisse); X-glucoside (75 mg/l; B-7250, BIOSYNTH, Staad, Suisse); cefsulodine (10 mg/l; C 4786, Sigma, St Quentin Fallavier, France) et différentes concentrations de l'inhibiteur O,O-Dimethyl p-nitrophenyl phosphate (Paraoxon® méthyl; CAS n° 950-35-6; Riedel-deHaën, St Quentin Fallavier, France ; 5, 10, 25 mg/l).

Les différents milieux de détection ainsi obtenus sont ensuite coulés en boîte de Petri.

Ensemencement des bactéries *Salmonella* : les boîtes de Petri sont inoculées, incubées et lues comme dans l'exemple 1.

Les résultats obtenus sont présentés dans le tableau 2.

**Tableau 2 : Effets de l'inhibiteur O,O-Dimethyl p-nitrophenyl phosphate sur l'activité des estérases libres dans un échantillon dans un échantillon lors de l'identification de bactéries Salmonella**

| | | [O,O-Dimethyl p-nitrophenyl phosphate] en mg/l | | | | | | | |
|---|---|---|---|---|---|---|---|---|---|
| | | 0 | | 5 | | 10 | | 25 | |
| N° souche | N° selle | Dépôt | Colonies | Dépôt | Colonies | Dépôt | Colonies | Dépôt | Colonies |
| aucune | C | 2 | 0 | 1 | 0 | 0,5 | 0 | 0 | 0 |
| | D | 2 | 0 | 1 | 0 | 1 | 0 | 0 | 0 |
| *Salmonella* | C | 2 | 0,5 | 1 | 0.5 | 0,5 | 1 | 0 | 0,5 |
| *Typhi* | D | 2 | 0,5 | 1 | 0.5 | 1 | 0,5 | 0 | 0,5 |
| *Salmonella* | C | 2 | 2 | 1 | 2 | 0,5 | 2 | 0 | 2 |
| *Enteritidis* | D | 2 | 2 | 1 | 2 | 1 | 2 | 0 | 2 |

Comme le présente le tableau 2, la coloration est diminuée au niveau du point de dépôt de la selle en présence de l'inhibiteur, alors qu'elle est maintenue au niveau des colonies des bactéries, que l'inhibiteur soit présent ou absent. Les enzymes libres dans les selles sont donc bien inhibées par l'inhibiteur, alors que les enzymes provenant des bactéries ne sont pas inhibées.
Ces résultats démontrent que les réactions enzymatiques aspécifiques dues à la présence d' « enzymes libres » sont inhibées fortement en présence de l'inhibiteur O,O-Dimethyl p-nitrophenyl phosphate, ce qui limite la détection de faux positifs.

### Exemple 3 : Inhibition des estérases libres dans un échantillon par le O,O-Di (2-chloroethyl) -O-(3-chloro-4-methylcoumarin-7-YL) phosphate lors de l'identification de bactéries salmonelles

Le but des expériences présentés dans cet exemple est de démontrer l'effet inhibiteur de O,O-Di (2-chloroethyl) -O-(3-chloro-4-methylcoumarin-7-YL) phosphate (Haloxon® ; Sigma, St Quentin Fallavier, France) sur des estérases libres dans un échantillon (selles) contaminé par des Salmonelles.

Préparation du milieu de détection : 250 ml du milieu de l'exemple 1 dans lequel le O,O-Diethyl p-nitrophenyl phosphate est remplacé par différentes concentrations de O,O-Di (2-chloroethyl) -0-(3-chloro-4-methylcoumarin-7-YL) phosphate (Haloxon® ; CAS n°321-55-1 ; R276995, Sigma, St Quentin Fallavier, France; 0 ; 1 ; 10 ; 100 ou 1000 mg/l) sont préparés selon le procédé de l'exemple 1.
Les différents milieux de détection ainsi obtenus sont ensuite coulés en boîte de Petri. Ensemencement des bactéries *Salmonella* : les boîtes de Petri sont inoculées, incubées et lues comme dans l'exemple 1.
Les résultats obtenus sont présentés dans le tableau 3.

**Tableau 3 : Effets de l'inhibiteur O,O-Di (2-chloroethyl) -O-(3-chloro-4-methylcoumarin-7-YL) phosphate sur l'activité des estérases libres dans un échantillon lors de l'identification de bactéries Salmonella**

| | | [O,O-Di (2-chloroethyl) -O-(3-chloro-4-methylcoumarin-7-YL) phosphate] en mg/l | | | | | | | | | |
|---|---|---|---|---|---|---|---|---|---|---|---|
| | | 0 | | 1 | | 10 | | 100 | | 1000 | |
| N° souche | N° selle | Dépôt | Colonies | Dépôt | Colonies | Dépôt | Colonies | Dépôt | Colonies | Dépôt | Colonies |
| absence | A | 2 | 0 | 1 | 0 | 1 | 0 | 0,5 | 0 | 0 | 0 |
| | B | 2 | 0 | 1 | 0 | 0.5 | 0 | 0 | 0 | 0 | 0 |
| *Salmonella Enteritidis* | A | 2 | 2 | 1 | 2 | 1 | 2 | 0,5 | 2 | 0 | 2 |
| | B | 0 | 2 | 0 | 2 | 0 | 2 | 0 | 2 | 0 | 2 |
| *Salmonella Paratyphi A* | A | 2 | 2 | 1 | 2 | 1 | 2 | 0,5 | 1 | 0 | 0,5 |
| | B | 0 | 2 | 0 | 2 | 0 | 2 | 0 | 1 | 0 | 0,5 |
| *Salmonella Typhi* | A | 2 | 1 | 1 | 1 | 1 | 1 | 0,5 | 1 | 0 | 1 |
| | B | 0 | 1 | 0 | 1 | 0 | 1 | 0 | 1 | 0 | 1 |

Comme le présente le tableau 3, la coloration est diminuée au niveau du point de dépôt de la selle en présence de l'inhibiteur, alors qu'elle est maintenue au niveau des colonies des bactéries, que l'inhibiteur soit présent ou absent. Les enzymes libres dans les selles sont donc bien inhibées par l'inhibiteur, alors que les enzymes provenant des bactéries ne sont pas inhibées. Ces résultats démontrent que les réactions enzymatiques aspécifiques dues à la présence d' « enzymes libres » sont inhibées fortement en présence de l'inhibiteur O,O-Di (2-chloroethyl) -O-(3-chloro-4-methylcoumarin-7-YL) phosphate, ce qui limite la détection de faux positifs.

### Exemple 4 : Inhibition des estérases libres dans un échantillon par le O,O-Diethyl p-nitrophenyl phosphate, ,O-Dimethyl p-nitrophenyl phosphate et le O,O-Di (2-chloroethyl) -O-(3-chloro-4-methylcoumarin-7-YL) phosphate lors de l'identification de levures

Le but des expériences présentées dans cet exemple est de démontrer l'effet inhibiteur des estérases libres du O,O-Diethyl p-nitrophenyl phosphate, ,O-Dimethyl p-nitrophenyl phosphate et le O,O-Di (2-chloroethyl) -O-(3-chloro-4-methylcoumarin-7-YL) phosphate sur des selles contaminées par des levures *Candida.*

Préparation du milieu de détection : un volume de 250 ml de milieu de détection est préparé à partir d'une poudre de milieu ayant la composition suivante :

| | |
|---|---|
| Peptones | 10 g/l |
| Glucose | 1g/l |
| Tris | 0,16 g/l |
| Agar | 14 g/l |

La fonte est réalisée à 100°C, et le milieu est autoclavé 15 minutes à 121°C. On ajoute ensuite les additifs dans le milieu : Magenta C8 (500mg/l; B-7102, BIOSYNTH, Staad, Suisse); X-glucoside (75 mg/l; B-7250, BIOSYNTH, Staad, Suisse); cefsulodine (10 mg/l; C 4786, Sigma, St Quentin Fallavier, France), ainsi que l'inhibiteur d'estérase :
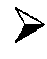 O,O-Diethyl p-nitrophenyl phosphate: 5 mg/l
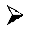 O-Dimethyl p-nitrophenyl phosphate: 25 mg/l ou
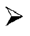 O,O-Di (2-chloroethyl) -O-(3-chloro-4-methylcoumarin-7-YL) phosphate: 75 mg/l
Les différents milieux de détection ainsi obtenus sont ensuite coulés en boîte de Petri.

Ensemencement des levures du genre *Candida* : 10 µl de selles sont déposés sur boîte de Petri, cette suspension de selle est mélangée ou non à 10µl d'une suspension de *Candida* (et isolée en 3 cadrans sur une boîte de Petri. Différentes selles et différentes souches du genre *Candida* provenant de la collection de la demanderesse sont utilisées selon ce protocole. Les boîtes de Petri sont incubées 24 heures à 37°C.
La lecture de l'apparition d'une coloration au niveau du point de dépôt de la selle et au niveau des colonies de *Candida* est effectuée selon une échelle semi-quantitative :
0 = absence de coloration
0,5 = trace de coloration
1 = coloration faible
2 = coloration forte .

Les résultats obtenus sont présentés dans le tableau 4.

**Tableau 4 : Effets des inhibiteurs O,O-Diethyl p-nitrophenyl phosphate, O,O-Dimethyl p-nitrophenyl phosphate, O,O-Di (2-chloroethyl) -O-(3-chloro-4-methylcoumarin-7-YL) phosphate sur l'activité des estérases libres dans un échantillon lors de l'identification de levures Candida**

| | | Sans inhibiteur | | O,O-Diethyl p-nitrophenyl phosphate: 5 mg/l | | O,O-Dimethyl p-nitrophenyl phosphate: 25 mg/l | | O,O-Di (2-chloroethyl) -O-(3-chloro-4-methylcoumarin-7-YL) phosphate : 75 mg/l | |
|---|---|---|---|---|---|---|---|---|---|
| N° souche | N° selle | Dépôt | Colonies | Dépôt | Colonies | Dépôt | Colonies | Dépôt | Colonies |
| absence | A | 2 | 0 | 0 | 0 | 0 | 0 | 0 | 0 |
| | B | 1 | 0 | 0 | 0 | 0 | 0 | 0 | 0 |
| *Candida albicans* | A | 2 | 1 | 1 | 1 | 1 | 1 | 1 | 1 |
| | B | 2 | 0.5 | 1 | 0.5 | 1 | 0.5 | 1 | 0.5 |
| *Candida dubliniensis* | A | 2 | 2 | 1 | 0.5 | 1 | 1 | 1 | 1 |
| | B | 2 | 1 | 1 | 0.5 | 1 | 1 | 1 | 1 |
| *Candida kruseï* | A | 2 | 1 | 1 | 0.5 | 1 | 0.5 | 1 | 0.5 |
| | B | 2 | 1 | 1 | 0.5 | 1 | 0.5 | 1 | 0.5 |

Comme le présente le tableau 4, la coloration est diminuée au niveau du point de dépôt de la selle en présence des différents inhibiteurs, alors qu'elle est maintenue au niveau des colonies des levures, que l'inhibiteur soit présent ou absent. Les enzymes libres dans les selles sont donc bien inhibées par l'inhibiteur, alors que les enzymes provenant des levures ne sont pas inhibées.
Ces résultats démontrent que les réactions enzymatiques aspécifiques dues à la présence d' « enzymes libres » sont inhibées fortement en présence des inhibiteurs O,O-Diethyl p-nitrophenyl phosphate, O,O-Dimethyl p-nitrophenyl phosphate , O,O-Di (2-chloroethyl) -O-(3-chloro-4-methylcoumarin-7-YL) phosphate, ce qui limite la détection de faux positifs.

### Exemple 5 : Inhibition de l'α-glucosidases libre dans un échantillon par la castanospermine lors de l'identification de bactéries

Le but des expériences présentées dans cet exemple est de démontrer l'effet inhibiteur de la castanospermine sur l'α-glucosidase libres dans un échantillon (selles) contaminé par des souches de *Staphylococcus aureus.*

Préparation du milieu de détection : 250 ml du milieu de l'exemple 1 dans lequel le O,O-Diethyl p-nitrophenyl phosphate est remplacé par différentes concentrations de castanospermine : 0 ; 1 ; 2 ; 4 et 8 g/l) sont préparés selon le procédé de l'exemple 1.
Les différents milieux de détection ainsi obtenus sont ensuite coulés en boîte de Petri.

Ensemencement des souches de *staphylococcus aureus:* les boîtes de Petri sont inoculées, incubées et lues comme dans l'exemple 1.
Les résultats obtenus sont présentés dans le tableau 6.

**Tableau 6 : Effets de la Castanospermine sur l'activité des enzymes α-glucosidase libres dans un échantillon lors de l'identification de souches de Staphylococcus aureus**

| | | [Castanospermine] en g/l | | | | | | | | | |
|---|---|---|---|---|---|---|---|---|---|---|---|
| | | 0 | | 1 | | 2 | | 4 | | 8 | |
| N° souche | N° selle | Dépôt | Colonies | Dépôt | Colonies | Dépôt | Colonies | Dépôt | Colonies | Dépôt | Colonies |
| absence | A | 2 | 0 | 1 | 0 | 1 | 0 | 0,5 | 0 | 0 | 0 |
| | B | 1 | 0 | 0.5 | 0 | 0.5 | 0 | 0 | 0 | 0 | 0 |
| *Staphylococcus aureus* n° 1 | A | 2 | 2 | 2 | 2 | 1 | 2 | 1 | 2 | 1 | 1 |
| *Staphylococcus aureus* n° 2 | B | 2 | 2 | 2 | 2 | 1 | 2 | 0,5 | 2 | 0 | 1 |

Comme le présente le tableau 6, la coloration est diminuée au niveau du point de dépôt de la selle en présence de la castanospermine, alors qu'elle est maintenue au niveau des colonies des bactéries, que l'inhibiteur soit présent ou absent. Les enzymes libres dans les selles sont donc bien inhibées par l'inhibiteur, alors que les enzymes provenant des bactéries ne sont pas inhibées.
Ces résultats démontrent que les réactions enzymatiques aspécifiques dues à la présence d' « enzymes libres » sont inhibées fortement en présence de l'inhibiteur castanospermine, ce qui limite la détection de faux positifs.

A noter qu'une variante aux exemples 1 à 6 consiste à ajouter l'inhibiteur dans le milieu de prélèvement au lieu de l'ajouter directement dans le milieu de culture.

### Exemple 6 : Méthode pour identifier des inhibiteurs d'au moins une deuxième enzyme selon l'invention

Cette méthode consiste à effectuer l'expérience des exemples 1 ou 2 en remplaçant :
i) le milieu de culture décrit (y compris les substrats enzymatiques) par celui approprié aux microorganismes recherchés
**ii)** les selles par l'échantillon dans lequel on recherche lesdits microorganismes et qui produit une réaction parasite avec un ou plusieurs des substrats enzymatiques inclus dans le milieu
**iii)** l'inhibiteur O,O-Diethyl p-nitrophenyl phosphate, ,O-Dimethyl p-nitrophenyl phosphate, O,O-Di (2-chloroethyl) -O-(3-chloro-4-methylcoumarin-7-YL) phosphate ou castanospermine par un inhibiteur potentiel à tester à différentes concentrations

## Revendications

1. Milieu de détection et/ou d'identification de microorganismes présents dans un échantillon, comprenant un milieu de culture et au moins un substrat qui peut être hydrolysé en un produit marqué par au moins une première enzyme non libre dans l'échantillon et, spécifique des microorganismes, **caractérisé en ce qu'**il comprend, en outre, au moins un inhibiteur d'au moins une deuxième enzyme, identique à celle-ci mais libre dans ledit échantillon et ne provenant pas d'un microorganisme, ladite première enzyme étant une estérase et ledit inhibiteur étant un composé de formule (I) 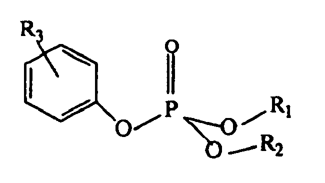 avec R1 est un atome d'hydrogène ou un groupement alkyle, aryle, halogène R2 est un atome d'hydrogène ou un groupement alkyle ou aryle, halogène R3 est rien ou un groupement alkyle, aryle, NO2

2. Milieu de détection et/ou d'identification, selon la revendication 1, **caractérisé en ce que** le microorganisme est une bactérie.

3. Milieu de détection et/ou d'identification, selon la revendication 2, **caractérisé en ce que** ladite bactérie appartient au genre *Salmonella.*

4. Milieu de détection et/ou d'identification, selon la revendication 1, **caractérisé en ce que** le microorganisme est une levure.

5. Milieu de détection et/ou d'identification, selon la revendication 4, **caractérisé en ce que** ladite levure appartient au genre *Candida.*

6. Milieu de détection et/ou d'identification selon l'une quelconques des revendications 1 a 5, **caractérisé en ce que** l'inhibiteur est le O,O-Diethyl p-nitrophenyl phosphate et/ou le O,O-Dimethyl p-nitrophenyl phosphate et/ou le O,O-Di (2-chloroethyl)-O-(3-chloro-4-methylcoumarin-7-YL) phosphate et/ou au moins un dérivé de ces molécules.

7. Milieu de détection et/ou d'identification selon la revendication 6, **caractérisé en ce que** la concentration en O,O-Diethyl p-nitrophenyl phosphate ou son dérivé dans le milieu de détection est comprise entre 0,1 et 15 mg/l, préférentiellement entre 1 et 10 mg/l.

8. Milieu de détection et/ou d'identification selon la revendication 6, **caractérisé en ce que** la concentration en O,O-Dimethyl p-nitrophenyl phosphate ou son dérivé dans le milieu de détection est comprise entre 0,1 et 100 mg/l, préférentiellement entre 10 et 50 mg/l.

9. Milieu de détection et/ou d'identification selon la revendication 6, **caractérisé en ce que** la concentration en O,O-Di(2-chloroethyl)-O-(3-chloro-4-methylcoumarin-7-YL) phosphate ou son dérivé dans le milieu de détection est comprise entre 1 et 1000 mg/l, préférentiellement entre 30 et 100 mg/l

10. Milieu de détection et/ou d'identification selon l'une quelconque des revendications 1 à 9 **caractérisé en ce que** ledit substrat est un substrat chromogène, préférentiellement un ester d' indoxyl ou de ses dérivés

11. Procédé détection et/ou d'identification de microorganismes comprenant :
• l'ensemencement des microorganismes à identifier sur un milieu de détection, selon l'une quelconque des revendications 1 à 10,
• l'incubation du milieu de détection ensemencé avec les microorganismes à identifier, et
• la détermination de la présence de microorganismes par la détection du ou des substrats hydrolysés en un produit marqué.

12. Utilisation du milieu de détection et/ou d'identification selon l'une quelconque des revendications 1 à 10 pour l'identification de microorganismes.

## Claims

1. Medium for detecting and/or identifying microorganisms present in a sample, comprising a culture medium and at least one substrate that can be hydrolysed to a labelled product by at least a first enzyme not free in the sample, and specific for said microorganisms, **characterised in that** it also comprises at least one inhibitor of at least a second enzyme, identical to the first enzyme but free in said sample and not originating from a microorganism, said first enzyme being an esterase and said inhibitor being a compound of formula (I) 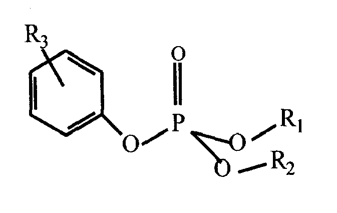 in which R1 is a hydrogen atom or an alkyl, aryl or halogen group,
R2 is a hydrogen atom, or an alkyl, aryl or halogen group,
R3 is nothing or an alkyl, aryl or NO2 group.

2. Detection and/or identification medium, according to claim 1, **characterised in that** the microorganism is a bacterium.

3. Detection and/or identification medium, according to claim 2, **characterised in that** said bacterium belongs to the *Salmonella* genus.

4. Detection and/or identification medium, according to claim 1, **characterised in that** the microorganism is a yeast.

5. Detection and/or identification medium, according to claim 4, **characterised in that** said yeast belongs to the *Candida* genus.

6. Detection and/or identification medium, according to claims 1 to 5, **characterised in that** the inhibitor is O,O-Diethyl p-nitrophenyl phosphate and/or O,O-Dimethyl p-nitrophenyl phosphate and/or O,O-Di (2-chloroethyl)-O-(3-chloro-4-methylcoumarin-7-YL) phosphate and/or at least one derivative of these molecules.

7. Detection and/or identification medium, according to claim 6, **characterised in that** the concentration of O,O-Diethyl p-nitrophenyl phosphate or its derivative in the detection medium is between 0,1 and 15 mg/l, preferably between 1 and 10 mg/l.

8. Detection and/or identification medium, according to claim 6, **characterised in that** the concentration of O,O-Dimethyl p-nitrophenyl phosphate or its derivative in the detection medium is between 0,1 and 100 mg/l, preferably between 10 and 50 mg/l.

9. Detection and/or identification medium, according to claim 6, **characterised in that** the concentration of O,O-Di (2-chloroethyl)-O-(3-chloro-4-methylcoumarin-7-YL) phosphate or its derivative in the detection medium is between 1 and 1 000 mg/l, preferably between 30 and 100 mg/l.

10. Detection and/or identification medium, according to claims 1 to 9, **characterised in that** said substrate is a chromogenic substrate, preferably an ester of indoxyl or of its derivatives.

11. Process for detecting and/or identifying microorganisms, comprising :
• seeding the microorganisms to be identified onto a detection medium according to claims 1 to 10,
• incubating the detection medium seeded with the microorganisms to be identified, and
• determining the presence of microorganisms by detecting the substrate(s) hydrolysed
• to a labelled product.

12. Use of a detection and/or identification medium according to claims 1 to 10, to identify microorganisms.

## Patentansprüche

1. Medium zum Nachweisen und/oder Identifizieren von in einer Probe befindlichen Mikroorganismen, umfassend ein Kulturmedium und mindestens ein Substrat, das von mindestens einem ersten Enzym, das in der Probe nicht frei befindlich ist, zu einem markierten Produkt hydrolisiert werden kann, und das spezifisch für die Mikroorganismen ist, **dadurch aekennzeichnet, daß** es weiterhin mindestens einen Hemmstoff für mindestens ein zweites Enzym, das mit jenem identisch ist, jedoch frei in der Probe vorliegt und nicht von einem Mikroorganismus stammt, umfaßt, wobei es sich bei dem ersten Enzym um eine Esterase handelt und bei dem Hemmstoff um eine Verbindung der Formel (I) handelt, 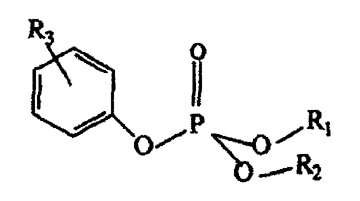 wobei R₁ ein Wasserstoffatom oder eine Alkyl-, Aryl- oder Halogengruppe bedeutet, R₂ ein Wasserstoffatom oder eine Alkyl- oder Aryl-Gruppe oder Halogen bedeutet, R₃ ohne Bedeutung ist oder eine Alkyl-, Aryl- oder NO₂-Grupp bedeutet.

2. Nachweis- und/oder Identifizierungsmedium nach Anspruch 1, **dadurch gekennzeichnet, daß** es sich bei dem Mikroorganismus um ein Bakterium handelt.

3. Nachweis- und/oder Identifizierungsmedium nach Anspruch 2, **dadurch gekennzeichnet, daß** das Bakterium zu der Gattung *Salmonella* gehört.

4. Nachweis- und/oder Identifizierungsmedium nach Anspruch 1, **dadurch gekennzeichnet, daß** es sich bei dem Mikroorganismus um eine Hefe handelt.

5. Nachweis- und/oder Identifizierungsmedium nach Anspruch 4, **dadurch gekennzeichnet, daß** die Hefe zu der Gattung *Candida* gehört.

6. Nachweis- und/oder ldentifizierungsmedium nach einem der Ansprüche 1 bis 5, **dadurch gekennzeichnet, daß** es sich bei dem Hemmstoff um O,O-Diethyl-p-nitrophenylphosphat und/oder O,O-Dimethyl-p-nitrophenylphosphat und/oder O,O-Di(2-chlorethyl)-O-(3-chlor-4-methylcumarin-7-yl)phosphat und/oder mindestens ein Derivat dieser Moleküle handelt.

7. Nachweis- und/oder Identifizierungsmedium nach Anspruch 6, **dadurch gekennzeichnet, daß** die Konzentration an O,O-Diethyl-p-nitrophenylphosphat oder seinem Derivat in dem Nachweismedium zwischen 0,1 und 15 mg/l, vorzugsweise zwischen 1 und 10 mg/l, liegt.

8. Nachweis- und/oder ldentifizierungsmedium nach Anspruch 6, **dadurch gekennzeichnet, daß** die Konzentration an O,O-Dimethyl-p-nitrophenylphosphat oder seinem Derivat in dem Nachweismedium zwischen 0,1 und 100 mg/l, vorzugsweise zwischen 10 und 50 mg/l, liegt.

9. Nachweis- und/oder ldentifizierungsmedium nach Anspruch 6, **dadurch gekennzeichnet, daß** die Konzentration an O,O-Di(2-chlorethyl)-O-(3-chlor-4-methylcumarin-7-yl)phosphat oder seinem Derivat in dem Nachweismedium zwischen 1 und 1000 mg/l, vorzugsweise zwischen 30 und 100 mg/l, liegt.

10. Nachweis- und/oder Identifizierungsmedium nach einem der Ansprüche 1 bis 9, **dadurch gekennzeichnet, daß** es sich bei dem Substrat um ein chromogenes Substrat, vorzugsweise um einen Indoxylester oder seine Derivate, handelt.

11. Verfahren zum Nachweisen und/oder Identifizieren von Mikroorganismen, das folgendes umfaßt:
• Beimpfen eines Nachweismediums nach einem der Ansprüche 1 bis 10 mit zu identifizierenden Mikroorganismen,
• Inkubieren des mit den zu identifizierenden Mikroorganismen beimpften Nachweismediums, und
• Bestimmen des Vorliegens von Mikroorganismen durch Nachweisen des zu einem markierten Produkt hydrolisierten Substrats bzw. der zu einem markierten Produkt hydrolisierten Substrate.

12. Verwendung des Nachweis- und/oder Identifizierungsmediums nach einem der Ansprüche 1 bis 10 zum Identifizieren von Mikroorganismen.
